(19) Europäisches Patentamt
European Patent Office
Office européen des brevets

(11) Veröffentlichungsnummer: **0 187 233**
**B1**

(12) # EUROPÄISCHE PATENTSCHRIFT

(45) Veröffentlichungstag der Patentschrift: **16.03.88**

(51) Int. Cl.⁴: **C 07 C 163/00**, A 61 K 31/165

(21) Anmeldenummer: **85114610.0**

(22) Anmeldetag: **16.11.85**

(54) **S-(Carbamoyl-phenylselenyl)-Derivate von Mercaptanen, Verfahren zu ihrer Herstellung und diese enthaltende pharmazeutische Präparate.**

(30) Priorität: **29.11.84 DE 3443467**

(43) Veröffentlichungstag der Anmeldung: **16.07.86 Patentblatt 86/29**

(45) Bekanntmachung des Hinweises auf die Patenterteilung: **16.03.88 Patentblatt 88/11**

(84) Benannte Vertragsstaaten: **AT BE CH DE FR GB IT LI LU NL SE**

(56) Entgegenhaltungen: **DE-A-3 027 075**

(73) Patentinhaber: **A. Nattermann & Cie. GmbH, Nattermannallee 1, D-5000 Köln 30 (DE)**

(72) Erfinder: **Dereu, Norbert, Dr., An der Holzhecke 11, D-5020 Frechen- Bachem (DE)**
Erfinder: **Wendel, Albrecht, Prof. Dr., Im Buckenloh 19, D-7400 Tübingen 1 (DE)**
Erfinder: **Sies, Helmut, Prof. Dr., Schillerstrasse 7, D-4000 Düsseldorf (DE)**
Erfinder: **Leyck, Sigurd, Dr., Am Quechenhauf 21, D-5024 Pulheim 2 (DE)**
Erfinder: **Römer, Axel, Dr., Heinrich- Imig- Strasse 16, D-5030 Hürth- Gleuel (DE)**
Erfinder: **Graf, Erich, Dr., Am Langen Hau 24, D-5014 Kerpen- Horrem (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

EP 0 187 233 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue S-(Carbamoyl-phenylselenyl)-Derivate von aliphatischen und aromatischen Mercaptanen, die sich durch wertvolle pharmakologische Eigenschaften auszeichnen, sowie Verfahren zu ihrer Herstellung und ihre Verwendung als Wirkstoffe in Arzneimitteln. Sie können besonders zur Behandlung von Krankheiten Verwendung finden, die durch eine Zellschädigung aufgrund vermehrter Bildung von aktiven Sauerstoffmetaboliten hervorgerufen werden, wie z. B. Leberschäden, Herzinfarkt, Entzündungen, Strahlenschäden.

Die erfindungsgemäßen Verbindungen entsprechen der allgemeinen Formel I

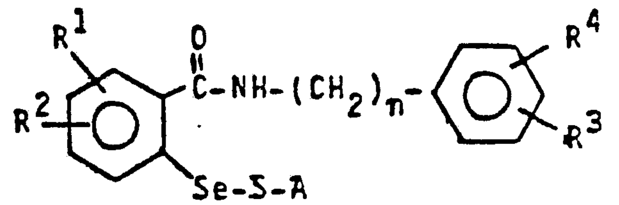

I

worin

R¹, R², R³, R⁴ gleich oder verschieden sind und unabhängig voneinander Wasserstoff, Halogen, $C_{1-4}$-Alkyl, $C_{1-3}$-Alkoxy, Hydroxy, Trifluormethyl, Nitro, Cyan, Carboxy, $C_{1-2}$-Alkoxycarbonyl, Carboxy-$C_{1-4}$-alkyl, $C_{1-2}$-Alkoxycarbonyl-$C_{1-4}$-alkyl bedeuten und

A für eine gerade oder verzweigte Alkylgruppe mit 1-4 Kohlenstoffatomen steht, die 1-3mal durch Carboxy, Hydroxy, Mercapto, Carboxyalkylcarbamoyl substituiert sein kann, wobei Veresterung der funktionell modifizierbaren Carboxylgruppe mit einem $C_1$-$C_3$-Alkohol möglich ist oder eine Phenyl-, Carboxyphenyl-, Alkoxycarbonylphenyl-, Pyridyl- oder Pyridylalkylgruppe bedeutet und

n für Null oder eins steht.

Halogen bedeutet Fluor, Chlor, Brom. Als Alkylreste mit 1-4 Kohlenstoffatomen seien Methyl, Ethyl, Propyl, Isopropyl, Butyl, Isobutyl, sec.-Butyl, tert.-Butyl genannt, als Alkoxyreste mit 1-3 Kohlenstoffatomen kommen Methoxy, Ethoxy, Propoxy in Betracht.

Bevorzugt sind dabei Verbindungen, in denen $R^1$, $R^2$, $R^3$, $R^4$ gleich oder verschieden sind und unabhängig voneinander Wasserstoff, Fluor, Chlor, Methyl, Methoxy, Hydroxy, Trifluormethyl, Cyan, Carboxy, $C_{1-2}$-Alkoxycarbonyl, Carboxy-$C_{1-4}$-alkyl, $C_{1-2}$-Alkoxycarbonyl-$C_{1-4}$-alkyl oder Nitro bedeuten. Besonders bevorzugt sind dabei Verbindungen, in denen $R^1$, $R^2$ gleich oder verschieden sind und unabhängig voneinander Wasserstoff, Fluor, Chlor, Methyl, Methoxy, Hydroxy, Trifluormethyl oder Nitro bedeuten, während $R^3$, $R^4$ für Wasserstoff, Methoxy oder Hydroxy stehen.

A entspticht dem Rest der für die jeweilige Umsetzung verwendeten Mercaptoverbindung wie z. B. Ethylmercaptan, Mercaptoessigsäure, 2-Mercaptopropionsäure, 3-Mercaptopropionsäure, Mercaptoessigsäuremethylester, Mercaptoessigsäureethylester, 2-Mercaptopropionsäuremethylester, 3-Mercaptopropionsäuremethylester, 2-Mercaptopropionylglycin, 2-Mercaptopropionylglycinethylester, Mercaptobernsteinsäure, 3-Mercapto-1,2-propandiol, threo-1,4-Dimercapto-2,3-butandiol, Thiophenol, Thiosalicylsäure, Thiosalicylsäuremethylester, 2-Mercaptopyridin, 3-Mercaptomethylpyridin.

Verbindungen der Formel I, die ein Chiralitätszentrum besitzen, können je nach Art der verwendeten Ausgangssubstanzen als Racemate oder in Form der D- oder L-Enantiomeren vorliegen. Falls eine Trennung der Racemate erwünscht ist, wird diese zweckmäßigerweise nach an sich bekannten Verfahren mit geeigneten optisch aktiven Basen über die Bildung diastereomerer Salze oder durch Chromatographie an optisch aktivem Säulenmaterial durchgeführt.

Erfindungsgemäße Verbindungen sind beispielsweise:

S-(2-Phenylcarbamoyl-phenylselenyl)-ethylmercaptan
S-[2-(2-Fluorphenylcarbamoyl)-phenylselenyl]-ethyl-mercaptan
S-(2-Phenylcarbamoyl-phenylselenyl)-mercaptoessigsäure-ethylester
S-(2-Phenylcarbamoyl-phenylselenyl)-3-mercaptopropionsäure
S-(2-Phenylcarbamoyl-6-methoxy-phenylselenyl)-3-mercapto-propionsäure
S-(2-Phenylcarbamoyl-phenylselenyl)-DL-2-mercaptopropionylglycin
S-(2-Phenylcarbamoyl-phenylselenyl)-DL-2-mercaptopropinylglycinethylester
S-[2-(3-Fluorphenylcarbamoyl)-phenylselenyl]-DL-2-mercaptopropionylglycin
S-[2-(4-Methoxyphenylcarbamoyl)-phenylselenyl]-DL-2-mercaptopropionylglycin
S-(2-Phenylcarbamoyl-5-chlor-phenylselenyl)-DL-2-mercaptopropionylglycin
S-(2-Phenylcarbamoyl-6-methoxy-phenylselenyl)-DL-2-mercaptopropionylglycin
S-[2-(4-Nitrophenylcarbamoyl)-phenylselenyl]-DL-2-mercaptopropionylglycin
S-[2-(4-Chlorphenylcarbamoyl)-phenylselenyl]-DL-2-mercaptopropionylglycin
S-(2-Phenylcarbamoyl-phenylselenyl)-3-mercaptopropionylglycin

S-(2-Phenylcarbamoyl-phenylselenyl)-3-mercaptopropionyl-glycinethylester
S-(2-Phenylcarbamoyl-6-methoxy-phenylselenyl)-3-mercaptopropionylglycin
S-[2-(4-Nitrophenylcarbamoyl)-phenylselenyl]-3-mercaptopropionylglycin
S-(2-Phenylcarbamoyl-phenylselenyl)-DL-mercaptobernsteinsäure
S-[2-(3-Fluorphenylcarbamoyl)-phenylselenyl]-DL-mercaptobernsteinsäure
S-[2-(4-Methoxyphenylcarbamoyl)-phenylselenyl]-DL-mercaptobernsteinsäure
S-(2-Phenylcarbamoyl-5-chlor-phenylselenyl)-DL-mercaptobernsteinsäure
S-(2-Phenylcarbamoyl-6-methoxy-phenylselenyl)-DL-mercaptobernsteinsäure
S-[2-(4-Nitrophenylcarbamoyl)-phenylselenyl]-DL-mercaptobernsteinsäure
S-[2-(4-Chlorphenylcarbamoyl)-phenylselenyl]-DL-mercaptobernsteinsäure
S-[2-(4-Chlorphenylcarbamoyl)-6-methoxy-phenylselenyl]-DL-mercaptobernsteinsäure
S-[2-(4-Nitrophenylcarbamoyl)-6-methoxy-phenylselenyl]-DL-mercaptobernsteinsäure
S-[2-(3,4-Dichlorphenylcarbamoyl)-6-methoxy-phenylselenyl]-DL-mercaptobernsteinsäure
S-[2-(3,4-Difluorphenylcarbamoyl)-6-methoxy-phenylselenyl]-DL-mercaptobernsteinsäure
S-[2-(3-Chlor-4-methoxy-phenylcarbamoyl)-6-methoxy-phenylselenyl]-DL-mercaptobernsteinsäure
S-[2-(3,4-Dimethoxyphenylcarbamoyl)-6-methoxy-phenylselenyl]-DL-mercaptobernsteinsäure
S-[2-(4-Chlor-3-fluor-phenylcarbamoyl)-6-methoxy-phenylselenyl]-DL-mercaptobernsteinsäure
S-[2-(4-Methoxy-3-methyl-phenylcarbamoyl)-6-methoxy-phenylselenyl]-DL-mercaptobernsteinsäure
S-[2-(4-Methoxy-2-nitro-phenylcarbamoyl)-6-methoxy-phenylselenyl]-DL-mercaptobernsteinsäure
S-(2-Phenylcarbamoyl-phenylselenyl)-DL-threo-1,4-dimercapto-2,3-butandiol
S-(2-Benzylcarbamoyl-phenylselenyl)-mercaptoessigsäure
S-(2-Benzylcarbamoyl-6-methoxy-phenylselenyl)-mercaptoessigsäure
S-(2-Benzylcarbamoyl-6-methoxy-phenylselenyl)-DL-2-mercaptopropionsäure
S-(2-Benzylcarbamoyl-phenylselenyl)-DL-2-mercaptopropionylglycin
S-(2-Benzylcarbamoyl-6-methoxy-phenylselenyl)-DL-2-mercaptopropionylglycin
S-(2-Benzylcarbamoyl-phenylselenyl-)3-mercaptopropionylglycin
S-(2-Benzylcarbamoyl-6-methoxy-phenylselenyl)-3-mercaptopropionylglycin
S-(2-Phenylcarbamoyl-phenylselenyl)-thiophenol
S-[2-(4-Nitrophenylcarbamoyl)-phenylselenyl]-thiophenol
S-(2-Phenylcarbamoyl-phenylselenyl)-mercaptosalicylsäure
S-(2-Phenylcarbamoyl-phenylselenyl)-mercaptosalicylsäure-methylester
S-(2-Phenylcarbamoyl-phenylselenyl)-2-mercaptopyridin

Die erfindungsgemäßen Substanzen weisen glutathionperoxidaseartige Eigenschaften auf und sind damit in der Lage, dieses Enzym zu ersetzen und auf diese Weise im Zusammenwirken mit Mercaptanen (z. B. Glutathion) die schädigende Wirkung aktiver Sauerstoffmetaboliten zu verhindern.

Die selenabhängige Glutathion(GSH)-Peroxidase(Px) katalysiert die Reduktion von $H_2O_2$ und organischen Hydroperoxiden.

$$2\,GSH + H_2O_2 \xrightarrow{GSH\text{-}Px} GSSG + 2H_2O$$

$$2\,GSH + ROOH \xrightarrow{GSH\text{-}Px} GSSG + ROH + H_2O$$

Das selenhaltige Enzym schützt die Zellen gegen Peroxidation und spielt eine wichtige Rolle in der Modulierung des Arachidonsäure-Stoffwechsels (C.C.Reddy, E.J.Massaro, Fundam. and Appl. Toxicology (3), 9-10 (1983), S. 431-436 und L. Flohé in Free Radicals in Biology, Vol. V, Edited by W.A. Pryor 1982 Academic Press, S. 223-254).

Die Glutathion-Peroxidase spielt bei allen Erkrankungen eine Rolle, bei denen es zu einer Zellschädigung des betreffenden Gewebes und schließlich zur Nekrose aufgrund einer vermehrten Bildung von aktiven Sauerstoffmetaboliten in Form von Peroxiden (z. B. Lipidperoxide und Wasserstoffperoxid) kommt. Dieser sogenannte "oxidative Streß" wird z. B. beobachtet bei Lebererkrankungen - induziert durch entzündliche oder autoimmunologische Reaktionen, durch Alkohol oder durch Medikamente - aber auch bei anderen Erkrankungen, wie z. B. Herzinfarkt. Es ist bekannt, daß nach einem Herzinfarkt in das geschädigte Gebiet Leukozyten einwandern und der Zelluntergang von einer wermehrten Freisetzung der genannten aktiven Sauerstoffmetaboliten begleitet ist. Dies führt schließlich zu einem fortschreitenden Gewebeabbau.

In solchen Fällen ist das hierfür wichtige und natürlicherweise vorhandene Schutzsystem, bestehend aus verschiedenen, Peroxide und aktiven Sauerstoff abbauenden Enzymen, überfordert. Hierzu gehören Superoxiddismutase, Katalase, und besonders das Glutathion-Redox-System mit der dazu gehörenden Enzymkomponente Glutathion-Peroxidase. Gerade diesem letzteren Prinzip kommt eine große Bedeutung zu, da es sowohl organische Peroxide als auch Wasserstoffperoxid entgiften kann. Es ist belegt, daß dieses System für die intakte Leberfunktion eine große Rolle spielt (Wendel et al.: Biochemical Pharmacology, Vol. 31, S. 3601 (1982)) und z. B. das Ausmaß eines experimentellen Leberschadens gerade von diesem System abhängt, d. h. von dem Gehalt der Leber an Glutathion einerseits und von der Aktivität des Enzyms Glutathion-Peroxidase andererseits. Im Verlauf einer allgemeinen Entzündung wird dieser Leberschutzmechanismus stark reduziert (Bragt et al., Agents and Actions, Supp. 17, S. 214 (1980), wodurch die Leber einen stark erhöhten "oxidativen Streß"erleidet.

Eine sehr wichtige Rolle spielen die reaktiven Sauerstoffmetaboliten als Mediatoren von Entzündungen. Sie scheinen sowohl bei Leucotaxis, Gefäßdurchlässigkeit, Bindegewebsschäden Psoriasis und Immunkomplex/Komplement-induzierten Effekten mitzuwirken als auch bei Schäden, wie sie durch Wiedereinströmen in ischämischen Bereichen entstehen (L. Flohé et al., in The Pharmacology of Inflammation, ed. IL. Bonta et al., Handbook of Inflammation, Vol. 5, Elsevier, Amsterdam, S. 255-270).

Auch die Schäden nach ionisierender Bestrahlung sind auf die Bildung von Radikalen und aktiven Sauerstoffmetaboliten zurückzuführen. Ein Weg für die chemische Zytoprotektion besteht somit in der Stärkung des Glutathion/Glutathionperoxidase-Systems.

Die Messung der Glutathion-Peroxidase-Aktivität erfolgte nach der Methode von A. Wendel (A. Wendel, Methods in Enzymology Vol. 77, 325-333 (1981)). Gemessen wird in diesem Versuch die Mercaptankonzentration mittels Ellmans Reagenz.

Als Reduktionsmittel diente in diesem Falle nicht Glutathion, sondern die mercaptanhaltige Substanz, die für die Synthese der jeweiligen Verbindung verwendet wurde. Es wurde nun überraschenderweise gefunden, daß die erfindungsgemäßen Verbindungen der Formel I eine glutathionperoxidaseartige Wirkung besitzen. Die Reaktion der Benzisoselenazolone mit Mercaptanen erfolgt am Beispiel des 2-Phenyl-1,2-benzisoselenazol-3(2H)-on nach folgender Gleichung

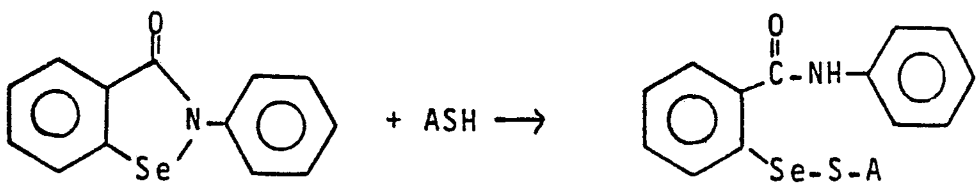

A = Mercaptanrest

**Glutathionperoxidaseartige Wirkung**

Bei in vitro-Untersuchungen wurde die Katalyse des Peroxidaseabbaus geprüft. Dabei wurde festgestellt, daß die erfindungsgemäßen Verbindungen die Glutathion-Peroxidase ersetzen können.

| | | |
|---|---|---|
| ROOH | Erfindungsgemäße→ Verbindungen | ROH |
| $H_2O_2$ | | $H_2O$ |
| RSH | | R-S-S-R |

Die Reaktionsgeschwindigkeiten wurden nach der Methode von A. Wendel ermittelt (A. Wendel, Methods in Enzymology Vol. 77, S. 325-333 (1981)). Als Referenzsubstanz wurde S-(2-Phenylcarbamoyl-phenylselenyl)-glutathion benutzt. In Gegenwart einer 1 mmolaren Konzentration an Glutathion wird mit tert.-Butylhydroperoxid eine Reaktionsgeschwindigkeit von 1,17 x $10^6$ Einheiten pro Mol beobachtet. Diese Aktivität wird zum besseren Vergleich als 100 % eingesetzt.

| | Katalytische Aktivität % |
|---|---|
| S-(2-Phenylcarbamoyl-phenylselenyl)-DL- threo-1,4-dimercapto-2,3-butandiol | 390 |
| S-(2-Phenylcarbamoyl-phenylselenyl)- thiophenol | 28 |
| S-(2-Phenylcarbamoyl-phenylselenyl)-3- mercaptopropionsäure | 47 |
| S-(2-Phenylcarbamoyl-phenylselenyl)- mercaptoessigsäureethylester | 330 |
| S-[2-(4-Nitrophenylcarbamoyl)-phenyl- selenyl]-thiophenol | 67 |
| S-(6-Methoxy-2-phenylcarbamoyl)-phenyl- selenyl-DL-threo-1,4-dimercapto-2,3-butandiol | 580 |

Die erfindungsgemäßen Verbindungen hemmen auch das durch den Cobra Venum Faktor induzierte Ödem der Rattenpfote.

Als Nachweis diente die Methode von S. Leyck, E. Etschenberg, V. Hadding und J. Winkelmann, Agents and Actions Vol. 13, 5/6 (1983).

Zur Ödemprovokation wird der Cobra Venum Faktor in einem Volumen won 0,1 ml Wasser subplantar an der linken Hinterpfote von Han-Wistar-Ratten beiderlei Geschlechts mit einem Körpergewicht von 150-200 g injiziert.

Die Bestimmung des Ödems erfolgt plethismographisch direkt vor und 3 Stunden nach Ödemprovokation. Die erhaltenen Differenzwerte der einzelnen Gruppen werden ermittelt und die prozentuale Änderung

gegenüber unbehandelten Kontrollen festgestellt. Die Ergebnisse sind in der folgenden Tabelle zusammengefaßt.

| Substanz | Entzündungshemmende Wirkung in % Dosis 100 mg/kg i.m. |
|---|---|
| S-(2-Phenylcarbamoyl-phenylselenyl)- ethylmercaptan | - 32 |
| S-(2-Phenylcarbamoyl-phenylselenyl)- DL-2-mercaptopropionylglycin | - 71 |
| S-(2-Phenylcarbamoyl-phenylselenyl)- DL-mercaptobernsteinsäure | - 84 |
| S-[2-(4-Nitrophenylcarbamoyl)- phenylselenyl]-thiophenol | - 20 |
| S-(2-Phenylcarbamoyl-phenylselenyl)- 3-mercaptopropionsäure | - 52 |
| S-(2-Phenylcarbamoyl-phenylselenyl)- thiophenol | - 25 |

Die Herstellung der erfindungsgemäßen Verbindungen erfolgt durch Umsetzung von 1,2-Benzisoselenazolonen der Formel II, die nach den Vorschriften von DE-OS 30 27 073, DE-OS 30 27 074 bzw. DE-OS 30 27 075 erhalten werden, mit Mercaptanen

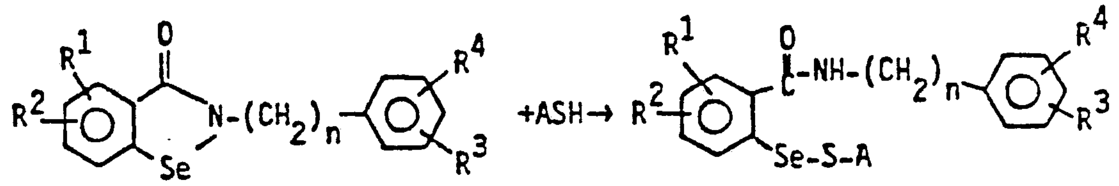


II I

Die Umsetzung mit dem jeweiligen Mercaptan erfolgt entweder in Suspensionen von 1,2-Benzisoselenazolonen in chlorierten Kohlenwasserstoffen wie Chloroform, Dichlormethan, Dichlorethan oder in einer Trifluoressigsäurelösung unter Rühren bei Raumtemperatur in 12-24 Stunden. Die für die Umsetzung verwendeten Mercaptane sind bekannte Verbindungen.

Die vorliegende Erfindung betrifft ebenfalls pharmazeutische Präparate, welche Verbindungen der Formel I enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z. B. Tabletten, Dragées, Kapseln, Suppositorien, Granulate, Lösungen, Emulsionen oder Suspensionen. Die Dosierung der Substanzen liegt üblicherweise zwischen 10 und 1000 mg pro Tag, vorzugsweise zwischen 30 und 300 mg pro Tag, und kann in einer Dosis oder mehreren Teildosen, vorzugweise in zwei bis drei Teildosen pro Tag, verabreicht werden.

Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind mit °C angegebenen und nicht korrigiert.

**Beispiel 1**

S-(2-Phenylcarbamoyl-phenylselenyl)-ethylmercaptan.

5 g (18,2 mmol) 2-Phenyl-1,2-benzisoselenazol-3(2H)-on werden in 150 ml Chloroform suspendiert (nur teilweise löslich). Zu dieser Suspension werden 2 g (32,2 mmol) Ethylmercaptan zugetropft. Man erhält eine klare Lösung, die über Nacht weitergerührt wird. Das Lösungsmittel wird eingedampft und der Rückstand mit 100 ml Hexan behandelt. Die so erhaltenen Kristalle werden abgesaugt und mit 50 ml Hexan nachgewaschen.

Ausbeute: 5,2 g (85 % d.Th.), Fp. 127 - 129°C

**Beispiel 2**

S-(2-Phenylcarbamoyl-phenylselenyl)-mercaptosalicylsäure.

Analog Beispiel 1 aus 5 g (18,2 mmol) 2-Phenyl-1,2-benzisoselenazol-3(2H)-on und 2,8 g (18,2 mmol) Mercaptosalicylsäure.

Ausbeute: 5 g (64,3 % d.Th.), Fp. 239 - 241°C

**Beispiel 3**

S-(2-Phenylcarbamoyl-phenylselenyl)-mercaptosalicylsäure-methylester.
Analog Beispiel 1 aus 5 g (18,2 mmol) 2-Phenyl-1,2-benzisoselenazol-3(2H)-on und 3,1 g (18,4 mmol) Mercaptosalicylsäuremethylester.
Ausbeute: 7,25 (90 % d.Th.), Fp. 138 - 140° C

**Beispiel 4**

S-(2-Phenylcarbamoyl-phenylselenyl)-3-mercaptopropionsäure.
Analog Beispiel 1 aus 5 g (18,2 mmol)
2-Phenyl-1,2-benzisoselenazol-3(2H)-on und 2 g (18,9 mmol) 3-Mercaptopropionsäure.
Ausbeute: 5,85 g (85 % d.Th.), Fp. 203 - 204° C

**Beispiel 5**

S-(2-Phenylcarbamoyl-phenylselenyl)-mercaptoessigsäure-ethylester.
Analog Beispiel 1 aus 2 g (7,3 mmol) 2-Phenyl-1,2-benzisoselenazol-3(2H)-on und 1 g (8,3 mmol) Mercaptoessigsäureethylester.
Ausbeute: 2,4 g (84 % d.Th.), Fp. 92 - 94° C

**Beispiel 6**

S-(2-Phenylcarbamoyl-phenylselenyl)-DL-2-mercaptopropionylglycin.
1 g (3,65 mmol) 2-Phenyl-1,2-benzisoselenazol-3(2H)-on und 0,6 g (3,68 mmol) DL-2-Mercaptopropionylglycin werden in 15 ml Trifluoressigsäure gelöst und 18 Stunden bei Raumtemperatur weitergerührt. Zu dieser Lösung werden dann 100 ml eines Eis-Wassergemisches hinzugegeben. Der ausgefallene Niederschlag wird mit Dichlormethan extrahiert. Nach Trocknen des Lösungsmittels und Eindampfen im Vakuum wird der Feststoff dann aus Ethanol/Wasser 7 : 3 umkristallisiert.
Ausbeute: 1,5 g (94 % d.Th.), Fp. 199° C

**Beispiel 7**

S-(2-Phenylcarbamoyl-phenylselenyl)-2-mercaptopyridin.
Analog Beispiel 1 aus 2,5 g (9,) mmol) 2-Phenyl-1,2-benzisoselenazol-3(2H)-on und 1,2 g (10,8 mmol) 2-Mercaptopyridin.
Ausbeute: 3,2 g (91,1 % d.Th.), Fp. 67° C (Zers.)

**Beispiel 8**

S-[2-(4-Nitrophenylcarbamoyl)-phenylselenyl] -thiophenol.
Analog Beispiel 1 aus 5 g (15,7 mmol) 2-(4-Nitrophenyl)-1,2-benzisoselenazol-3(2H)-on und 1,75 g (15,9 mmol) Thiophenol.
Ausbeute: 5,7 g (84,8 % d.Th.), Fp. 60° C (Zers.)

**Beispiel 9**

S-[2-(2-Fluorphenylcarbamoyl)-phenylselenyl]-ethylmercaptan.
Analog Beispiel 1 aus 5 g (17,1 mmol) 2-(3-Fluorphenyl)-1,2-benzisoselenazol-3(2H)-on und 2,2 g (35,5 mmol) Ethylmercaptan.
Ausbeute: 3,9 g (64,3 % d.Th.), Fp. 93 - 95° C

**Bespiel 10**

S-(2-Phenylcarbamoyl-phenylselenyl)-DL-mercaptobernsteinsäure.
Analog Beispiel 1 aus 1 g (3,65 mmol) 2-Phenyl-1,2-benzisoselenazol-3(2H)-on und 0,55 g (3,66 mmol) DL-Mercaptobernsteinsäure.
Ausbeute: 1,3 g (84 % d.Th.), Fp. 200° C (Zers.)

**Beispiel 11**

S-(2-Phenylcarbamoyl-phenylselenyl)-2-mercaptopropionylglycin.
Analog Beispiel 1 aus 2 g (7,3 mmol) 2-Phenyl-1,2-benzisoselenazol-3(2H)-on und 1,2 g (7,35 mmol) 3-Mercaptopropionylglycin.
Ausbeute: 3,0 g (94 % d.Th.), Fp. 199° C

**Beispiel 12**

S-(2-Benzylcarbamoyl-phenylselenyl)-mercaptoessigsäure.
Analog Beispiel 1 aus 2,88 g (10,0 mmol) 2-Benzyl-1,2-benzisoselenazol-3(2H)-on und 0,95 g (10,3 mmol) Mercaptoessigsäure.
Ausbeute: 0,95 g (25 % d.Th.), Fp. 145 - 147° C

**Beispiel 13**

S-(2-Benzylcarbamoyl-phenylselenyl)-3-mercapto-1,2-propandiol.
Analog Beispiel 1 aus 2,88 g (10,0 mmol) 2-Benzyl-1,2-benzisoselenazol-3(2H)-on und 1,1 g (10,2 mmol) 3-Mercapto-1,2-propandiol.
Ausbeute: 2,1 g (53 % d.Th.), Fp. 187° C

**Beispiel 14**

S-(2-Phenylcarbamoyl-phenylselenyl)-DL-threo-1,4-dimercapto-2,3-butandiol.
Analog Beispiel 6 aus 2,74 g (10 mmol) 2-Phenyl-1,2-benz-isoselenazol-3(2H)-on und 1,55 g (10 mmol) DL-threo-1,4-dimercapto-2,3-butandiol.
Die Reinigung der Substanz erfolgt über Säulenchromatographie.
Ausbeute: 0,5 g (11,6 % d.Th.), Fp. 78 - 82° C

**Beispiel 15**

S-(2-Phenylcarbamoyl-6-methoxy-phenylselenyl)-DL-2-mercaptopropionylglycin.
Analog Beispiel 1 aus 3,0 g (9,87 mmol) 7-Methoxy-2-phenyl-1,2-benzisoselenazol-3(2H)-on und 1,63 g (10 mmol) DL-2-Mercaptopropionylglycin.
Ausbeute: 4,5 g (97,6 % d.Th.), Fp. 242 - 245° C

**Beispiel 16**

S-(2-Phenylcarbamoyl-4-chlor-phenylselenyl)-ethylmercaptan.
Analog Beispiel 1 aus 3,0 g (9,72 mmol) 5-Chlor-2-phenyl-1,2-benzisoselenazol-3(2H)-on und 1 g (15,9 mmol) Ethylmercaptan.
Ausbeute: 3,1 g (86 % d.Th.), Fp. 163 - 166° C

**Patentansprüche** für die Vertragsstaaten BE, CH, DE, FR, GB, IT, LI, LU, NL, SE

1. S-(Carbamoyl-phenylselenyl)-Derivate von Mercaptanen der allgemeinen Formel I

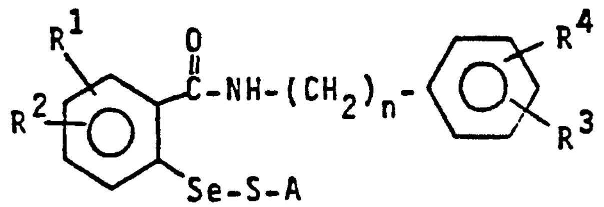

I

worin

$R^1$, $R^2$, $R^3$, $R^4$ gleich oder verschieden sind und unabhängig voneinander Wasserstoff, Halogen, $C_{1-4}$-Alkyl, $C_{1-3}$-Alkoxy, Trifluormethyl, Nitro, Cyan, Hydroxy, Carboxy, $C_{1-2}$-Alkoxycarbonyl, Carboxy-$C_{1-4}$-alkyl, $C_{1-2}$-Alkoxycarbonyl-$C_{1-4}$-alkyl bedeuten und

A für eine gerade oder verzweigte Alkylgruppe mit 1-4 Kohlenstoffatomen steht, die 1 bis 3mal durch Carboxy, Hydroxy, Mercapto, Carboxyalkylcarbamoyl substituiert sein kann, wobei Veresterung der funktionell modifizierbaren Carboxylgruppe mit $C_1$-$C_3$-Alkoholen möglich ist, oder eine Phenyl-, Carboxyphenyl-, Alkoxycarbonylphenyl-, Pyridyl- oder Pyridylalkylgruppe bedeutet und

n für Null oder eins steht.

2. S-(Carbamoyl-phenylselenyl)-Derivate von Mercaptanen gemäß Formel I, Anspruch 1, worin

$R^1$, $R^2$, $R^3$, $R^4$, gleich oder verschieden sind und unabhängig voneinander Wasserstoff, Fluor, Chlor, Methyl, Methoxy, Hydroxy, Trifluormethyl, Nitro, Cyan, Carboxy, $C_{1-2}$-Alkoxycarbonyl, Carboxy-$C_{1-4}$-alkyl, $C_{1-2}$-Alkoxycarbonyl-$C_{1-4}$-alkyl bedeuten und

A für eine gerade oder verzweigte Alkylgruppe mit 1-4 Kohlenstoffatomen steht, die 1 bis 3mal durch Carboxy, Hydroxy, Mercapto, Carboxyalkylcarbamoyl substituiert sein kann, wobei Veresterung der funktionell modifizierbaren Carboxylgruppe möglich ist, oder eine Phenyl-, Carboxyphenyl-, Alkoxycarbonylphenyl-, Pyridyl- oder Pyridylalkylgruppe bedeutet und

n für Null oder eins steht.

3. S-(Carbamoyl-phenylselenyl)-Derivate von Mercaptanen gemäß Formel I, Anspruch 1, worin

$R^1$, $R^2$ gleich oder verschieden sind und unabhängig voneinander Wasserstoff, Fluor, Chlor, Methyl, Methoxy, Hydroxy, Trifluormethyl, Nitro- und

$R^3$, $R^4$ gleich oder verschieden sind und unabhängig voneinander Wasserstoff, Fluor, Chlor, Methoxy, Hydroxy, Cyan, Carboxy, $C_{1-2}$-Alkoxycarbonyl, Carboxy-$C_{1-4}$-alkyl, $C_{1-2}$-Alkoxycarbonyl-$C_{1-4}$-alkyl bedeuten und

A für eine gerade oder verzweigte Alkylgruppe mit 1-4 Kohlenstoffatomen steht, die 1 bis 3mal durch Carboxy, Hydroxy, Mercapto, Carboxyalkylcarbamoyl substituiert sein kann, wobei Veresterung der funktionell modifizierbaren Carboxylgruppe möglich ist und

n für Null oder 1 steht.

4. S-(Carbamoyl-phenylselenyl)-Derivate von Mercaptanen gemäß Formel I, Anspruch 1 worin

$R^1$, $R^2$ gleich oder verschieden sind und unabhängig voneinander Wasserstoff, Fluor, Chlor, Methyl, Methoxy, Hydroxy, Trifluormethyl, Nitro und

$R^3$, $R^4$ gleich oder verschieden sind und unabhängig voneinander Wasserstoff, Fluor, Chlor, Methoxy, Hydroxy, Cyan, Carboxy, $C_{1-2}$-Alkoxycarbonyl, Carboxy-$C_{1-4}$-alkyl, $C_{1-2}$-Alkoxycarbonyl-$C_{1-4}$-alkyl bedeuten und

A eine Phenyl-, Carboxyphenyl-, Methoxycarbonylphenyl-, Pyridyl- oder Pyridylmethylgruppe bedeutet und

n für Null oder eins steht.

5. Verfahren zur Herstellung von Verbindungen der Formel I gemäß den Ansprüchen 1-4, dadurch gekennzeichnet, daß man in einem chlorierten Kohlenwasserstoff suspendiertes oder in Trifluoressigsäure gelöstes 1,2-Benzisoselenazolon der Formel II

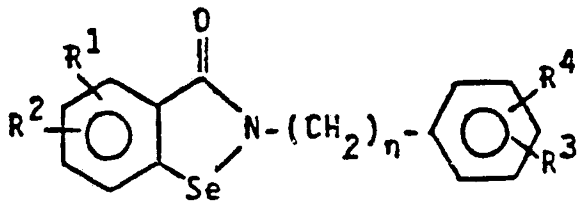

II

in der $R^1$, $R^2$, $R^3$, $R^4$ die in Formel I angegebenen Bedeutungen haben, mit einem Mercaptan der Formel III

A - SH III

unter Rühren bei Raumtemperatur in 12-24 Stunden umsetzt.

6. Pharmazeutische Präparate, dadurch gekennzeichnet, daß sie eine Verbindung der Formel I gemäß den Ansprüchen 1-4 als Wirkstoff im Gemisch mit üblichen pharmazeutischen Hilfs- und Trägerstoffen enthalten.

**Patentanspruch** für den Vertragsstaat AT:

Verfahren zur Herstellung von Verbindungen der allgemeinen Formel I

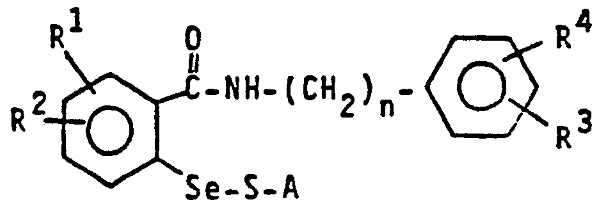

I

worin

$R^1$, $R^2$, $R^3$, $R^4$ gleich oder verschieden sind und unabhängig voneinander Wasserstoff, Halogen, $C_{1-4}$-Alkyl, $C_{1-3}$-Alkoxy, Trifluormethyl, Nitro, Cyan, Hydroxy, Carboxy, $C_{1-2}$-Alkoxycarbonyl, Carboxy-$_{1-4}$-alkyl, $C_{1-2}$-Alkoxycarbonyl-$C_{1-4}$-alkyl bedeuten und

A für eine gerade oder verzweigte Alkylgruppe mit 1 - 4 Kohlenstoffatomen steht, die 1 bis 3 mal durch Carboxy, Hydroxy, Mercapto, Carboxyalkylcarbamoyl substituiert sein kann, wobei Veresterung der funktionell modifizierbaren Carboxylgruppen mit $C_1$-$C_3$-Alkoholen möglich ist, oder eine Phenyl-, Carboxyphenyl-, Alkoxycarbonylphenyl-, Pyridyl- oder Pyridylalkylgruppe bedeutet und

n für null oder eins steht,

dadurch gekennzeichnet, daß man in einem chlorierten Kohlenwasserstoff suspendiertes oder in Trifluoressigsäure gelöstes 1,2-Benzisoselenazolon der Formel II

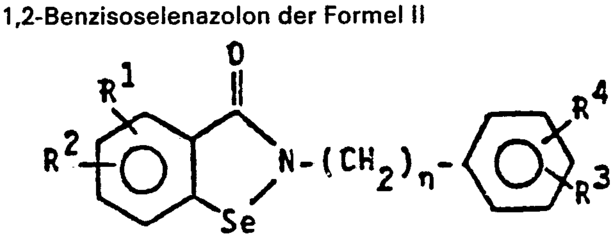

II

in der $R^1$, $R^2$, $R^3$, $R^4$ die in Formel I angegebenen Bedeutungen haben, mit einem Mercaptan der Formel III

A - SH III

unter Rühren bei Raumtemperatur in 12 - 24 Stunden umsetzt.

**Claims** for the Constructing States BE, CH, DE, FR, GB, IT, LI, LU, NL, SE

1. S-(Carbamoyl-phenylselenyl) derivatives of mercaptanes of the general formula I

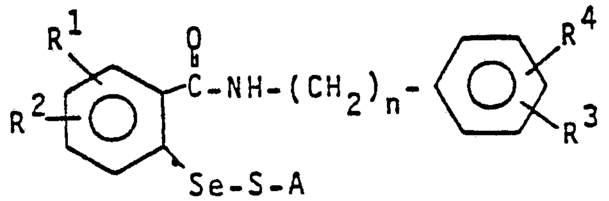

(I)

wherein

$R^1$, $R^2$, $R^3$, $R^4$ are identical or different and represent independently hydrogen, halogen, $C_{1-4}$-alkyl, $C_{1-3}$-alkoxy, trifluoromethyl, nitro, cyano, hydroxy, carboxy, $C_{1-2}$-alkoxycarbonyl, carboxy-$C_{1-4}$-alkyl, $C_{1-2}$-alkoxycarbonyl-$C_{1-4}$-alkyl and

A represents a straight or branched alkyl group having 1 to 4 carbon atoms which can be substituted by 1 to 3 carboxy, hydroxy, mercapto, carboxyalkylcarbamoyl groups, wherein in the functional modifiable carboxy group may be esterified with a $C_1$-$C_3$-alcohol, or a phenyl, carboxyphenyl, alkoxycarbonylphenyl, pyridyl or pyridylalkyl group and

n is zero or one.

2. S-(Carbamoyl-phenylselenyl) derivatives of mercaptanes according to formula I in claim 1, wherein

$R^1$, $R^2$, $R^3$, $R^4$ are identical or different and represent independently hydrogen, fluorine, chlorine, methyl, methoxy, hydroxy, trifluoromethyl, nitro, cyano, carboxy, $C_{1-2}$-alkoxycarbonyl, carboxy-$C_{1-4}$-alkyl, $C_{1-2}$-alkoxycarbonyl-$C_{1-4}$-alkyl and

A represents a straight or branched alkyl group having 1 to 4 carbon atoms which can be substituted by 1 to 3 carboxy, hydroxy, mercapto, carboxyalkylcarbamoyl groups, wherein the functional modifiable carboxy group may be esterified, or a phenyl, carboxyphenyl, alkoxycarbonylphenyl, pyridyl or pyridylalkyl group and

n is zero or one.

3. S-(Carbamoyl-phenylselenyl) derivatives of mercaptanes according to formula I in claim 1, wherein

$R^1$, $R^2$ are identical or different and independently represent hydrogen, fluorine, chlorine, methyl, methoxy, hydroxy, trifluoromethyl, nitro and

$R^3$, $R^4$ are identical or different and independently represent hydrogen, fluorine, chlorine, methoxy, hydroxy, cyano, carboxy, $C_{1-2}$-alkoxycarbonyl, carboxy-$C_{1-4}$-alkyl, $C_{1-2}$-alkoxycarbonyl-$C_{1-4}$-alkyl and

A represents a straight or branched alkyl group having 1 to 4 carbon atoms which can be substituted by 1 to 3 carboxy, hydroxy, mercapto, carboxyalkylcarbamoyl groups, wherein the functional modifiable carboxy group may be esterified and

n is zero or one.

4. S-(Carbamoyl-phenylselenyl) derivatives of mercaptanes according to formula I in claim 1, wherein

$R^1$, $R^2$ are identical or different and independently represent hydrogen, fluorine, chlorine, methyl, methoxy, hydroxy, trifluoromethyl, nitro and

$R^3$, $R^4$ are identical or different and independently represent hydrogen, fluorine, chlorine, methoxy, hydroxy, cyano, carboxy, $C_{1-2}$-alkoxycarbonyl, carboxy-$C_{1-4}$-alkyl, $C_{1-2}$-alkoxycarbonyl-$C_{1-4}$-alkyl and

A represents a phenyl, carboxyphenyl, methoxycarbonylphenyl, pyridyl or pyridylmethyl group and

n is zero or one.

5. A process for the preparation of compounds of formula I according to any of claims 1 to 4, characterized in that an 1,2-benzisoselenazolone of the formula

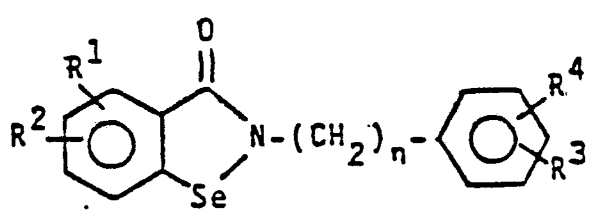

(II)

wherein

$R^1$, $R^2$, $R^3$, $R^4$ have the meanings given in formula I, suspended in a chlorinated hydrocarbon or

dissolved in trifluoroacetic acid, is reacted with a mercaptane of the formula III

A - SH III

at room temperature while stirring for 12 to 24 hours.

6. Pharmaceutical preparations, characterized in that they contain a compound of formula I according to any of claims 1 to 4 as active component in mixture with common pharmaceutical excipients and carriers.

**Claim** for contracting state AT

Process for the preparation of compounds of formula I

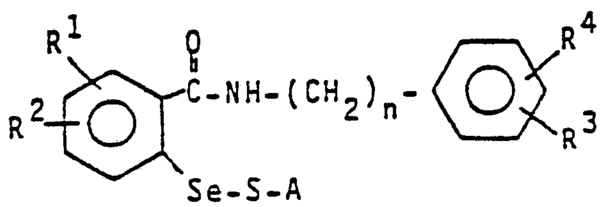

(I)

wherein

$R^1$, $R^2$, $R^3$, $R^4$ are identical or different and represent independently hydrogen, halogen, $C_{1-4}$-alkyl $C_{1-3}$-alkoxy, trifluoromethyl, nitro, cyano, hydroxy, carboxy, $C_{1-2}$-alkoxycarbonyl, carboxy-$C_{1-4}$-alkyl, $C_{1-2}$-alkoxycarbonyl-$C_{1-4}$-alkyl and

A represents a straight or branched alkyl group having 1 to 4 carbon atoms which can be substituted by 1 to 3 carboxy, hydroxy, mercapto, carboxyalkylcarbamoyl groups, wherein the functional modifiable carboxy group may be esterified with a $C_1$-$C_3$-alcohol, or a phenyl, carboxyphenyl, alkoxycarbonylphenyl, pyridyl or pyridylalkyl group and

n is zero or one,

characterized in that a 1,2-benzisoselenazolone of the formula II

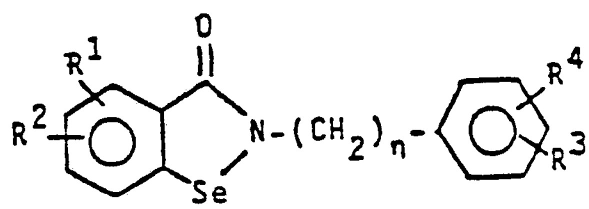

(II)

wherein

$R^1$, $R^2$, $R^3$, $R^4$ have the meanings given in formula I, suspended in a chlorinated hydrocarbon or dissolved in trifluoroacetic acid, is reacted with a mercaptane of the formula III

A - SH (III)

at room temperature while stirring for 12 to 24 hours.

**Revendications** pour les Etats Contractants BE, CH, DE, FR, GB, IT, LI, LU, NL, SE

1. Dérivés S-(carbamoyl-phénylsélényliques) de mercaptans de formule générale I

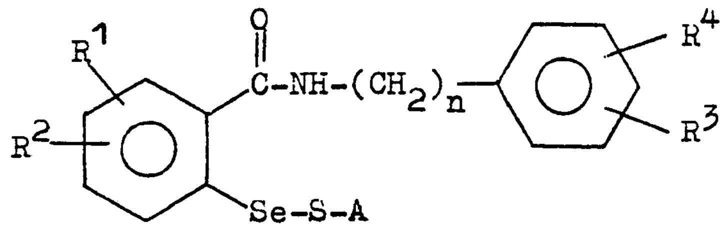

I

dans laquelle

$R^1$, $R^2$, $R^3$, $R^4$, ayant des significations identiques ou différentes, représentent chacun, indépendamment les uns des autres, l'hydrogène, un halogène, un groupe alkyle en C 1-C 4, alcoxy en C 1-C 3, trifluorométhyle, nitro, cyano, hydroxy, carboxy, (alcoxy en C 1-C 2)-carbonyle, carboxy-(alkyle en C 1-C 4), (alcoxy en C 1-C 2)-carbonyl-alkyle en C 1-C 4, et

A représente un goupe alkyle droit ou ramifié en C 1-C 4 qui peut porter un à trois substituants choisis parmi les groupes carboxy, hydroxy, mercapto, carboxyalkylcarbamoyle, l'estérification du groupe carboxyle apte à une modification fonctionnelle par des alcools en C 1 -C 3 étant possible, ou un groupe phényle, carboxyphényle, alcoxycarbonylphényle, pyridyle ou pyridylalkyle, et

n est égal à 0 ou 1.

2. Dérivés S-(carbamoyl-phénylsélényliques) de mercaptans de formule I, revendication 1,

dans laquelle

$R^1$, $R^2$, $R^3$, $R^4$, ayant des significations identiques ou différentes, représentent chacun, indépendamment les uns des autres, l'hydrogène, le fluor, le chlore, un groupe méthyle, méthoxy, hydroxy, trifluorométhyle, nitro, cyano, carboxy, (alcoxy en C 1-C 2)-carbonyle, carboxy-(alkyle en C 1-C 4), (alcoxy en C 1-C 2) carbonyl-alkyle en C 1-C 4, et

A représente un groupe alkyle droit ou ramifié en C 1-C 4 qui peut porter un à trois substituants choisis parmi les groupes carboxy, hydroxy, mercapto, carboxyalkylcarbamoyle, l'estérification du groupe carboxyle apte à une modification fonctionnelle étant possible, ou un groupe phényle, carboxyphényle, alcoxycarbonylphényle, pyridyle ou pyridylalkyle, et

n est égal à 0 ou 1.

3. Dérivés S-(carbamoyl-phénylsélényliques) de mercaptans de formule I, revendication 1 dans laquelle

$R^1$, $R^2$ ayant des significations identiques ou différentes, représentent chacun, indépendamment l'un de l'autre, l'hydrogène, le fluor, le chlore, un groupe méthyle, méthoxy, hydroxy, trifluorométhyle, nitro, et

$R^3$, $R^4$, ayant des significations identiques ou différentes, représentent chacun, indépendamment l'un de l'autre, l'hydrogène, le fluor, le chlore, un groupe méthoxy, hydroxy, cyano, carboxy, (alcoxy en C 1-C 2)-carbonyle, carboxy-(alkyle en C 1-C 4, (alcoxy en C 1-C 2)-carbonyl-alkyle en C 1-C 4, et

A représente un groupe alkyle droit ou ramifié en C 1-C 4 qui peut porter un à trois substituants choisis parmi les groupes carboxy, hydroxy, mercapto, carboxyalkylcarbamoyle, l'estérification du groupe carboxyle apte à une modification fonctionnelle étant possible, et

n est égal à 0 ou 1.

4. Dérivés S-(carbamoyl-phénylsélényliques) de mercaptans de formule I, revendication 1 dans laquelle

$R^1$, $R^2$, ayant des significations identiques ou différentes, représentent chacun, indépendamment l'un de l'autre, l'hydrogène, le fluor, le chlore, un groupe méthyle, methoxy, hydroxy, trifluorométhyle, nitro, et

$R^3$, $R^4$, ayant des significations identiques ou différentes, représentent chacun, indépendamment l'un de l'autre, l'hydrogène, le fluor, le chlore, un groupe méthoxy, hydroxy, cyano, carboxy, (alcoxy en C 1-C 2)-carbonyle, carboxy-(alkyle en C 1-C 4), (alcoxy en C 1-C 2-carbonyl-alkyle en C 1-C 4, et

A représente un groupe phényle, carboxyhényle, méthoxycarbonylphényle, pyridyle ou pyridylméthyle, et

n est égal à 0 ou 1.

5. Procédé de préparation des composés de formule I selon les revendications 1 à 4, caractérisé en ce que l'on fait réagir une 1,2-benzoisosélénazolone de formule II

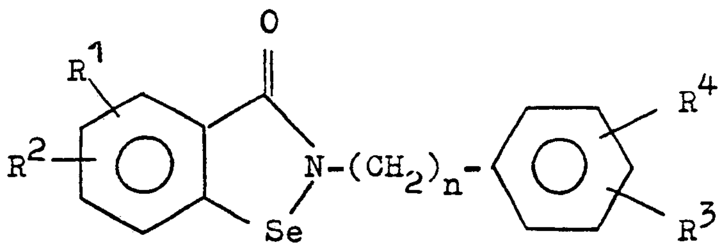

II

dans laquelle $R^1$, $R^2$, $R^3$, $R^4$ ont les significations indiquées en référence à la formule I, en suspension dans un hydrocarbure chloré ou en solution dans l'acide trifluoracétique, avec un mercaptan de formule III

A-SH III

sous agitation, à température ambiante, en une durée de 12 à 24 heures.

6. Compositions pharmaceutiques, caractérisées en ce qu'elles contiennent en tant que substance active un composé de formule I selon les revendications 1 à 4, en mélange avec des produits auxiliaires et véhicules pharmaceutiques usuels.

**Revendication** pour l'Etat Contractant AT

Procédé de préparation des composés de formule générale I

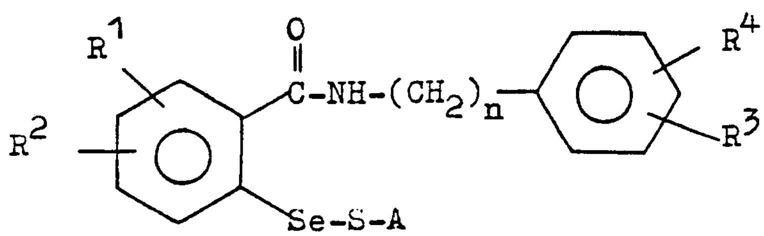

I

dans laquelle

$R^1$, $R^2$, $R^3$, $R^4$, ayant des significations identiques ou différentes, représentent chacun, indépendamment les uns des autres, l'hydrogène, un halogène, un groupe alkyle en C 1-C 4, alcoxy en C 1-C 3, trifluorométhyle, nitro, cyano, hydroxy, carboxy, (alcoxy en C 1-C 2)-carbonyle, carboxy-(alkyle en C 1-C 4), (alcoxy en C 1-C 2)-carbonyl-alkyle en C 1-C 4, et

A représente un groupe alkyle droit ou ramifié en C 1-C 4 qui peut porter un à trois substituants choisis parmi les groupes carboxy, hydroxy, mercapto, carboxyalkylcarbamoyle, l'estérification des groupes carboxyle aptes à une modification fonctionnelle par des alcools en C 1-C 3 étant possible, ou un groupe phényle, carboxyphényle, alcoxycarbonylphényle, pyridyle ou pyridylalkyle, et

n est égal à 0 ou 1.

caractérisé en ce que l'on fait réagir une 1,2-benzoisosélénazolone de formule II

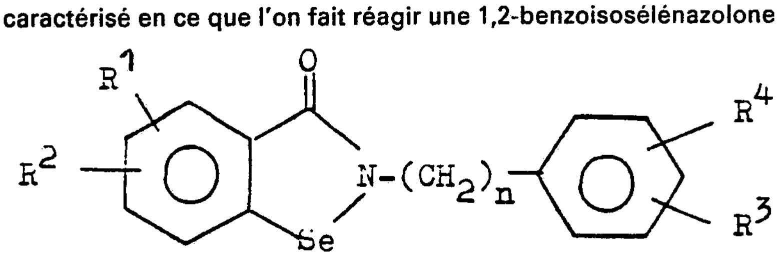

II

dans laquelle $R^1$, $R^2$, $R^3$, $R^4$ ont les significations indiquées en référence à la formule I, en suspension dans un hydrocarbure chloré ou en solution dans l'acide trifluoracétique, avec un mercaptan de formule III

A-SH III

sous agitation, à température ambiante, en des durées de 12 à 24 heures.